(19) Europäisches Patentamt
European Patent Office
Office européen des brevets

(11) Veröffentlichungsnummer : **0 173 152**
**B1**

(12) # EUROPÄISCHE PATENTSCHRIFT

(45) Veröffentlichungstag der Patentschrift : **28.09.88**

(51) Int. Cl.$^4$ : **C 07 D319/20**, A 01 N 47/34

(21) Anmeldenummer : **85110064.4**

(22) Anmeldetag : **10.08.85**

(54) **Benzoylharnstoffe.**

(30) Priorität : **24.08.84 DE 3431219**

(43) Veröffentlichungstag der Anmeldung : **05.03.86 Patentblatt 86/10**

(45) Bekanntmachung des Hinweises auf die Patenterteilung : **28.09.88 Patentblatt 88/39**

(84) Benannte Vertragsstaaten : **AT BE CH DE FR GB IT LI NL**

(56) Entgegenhaltungen :
**EP-A- 0 042 533**
**EP-A- 0 093 977**
**EP-A- 0 097 862**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Sirrenberg, Wilhelm, Dr.**
**Wuppertaler Strasse 21**
**D-4322 Sprockhövel (DE)**
Erfinder : **Marhold, Albrecht, Dr.**
**Carl-Duisberg-Strasse 329**
**D-5090 Leverkusen (DE)**
Erfinder : **Becker, Benedikt, Dr.**
**Metzkausener Strasse 14**
**D-4020 Mettmann (DE)**

**EP 0 173 152 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte 1-Phenyl-3-benzoyl-(thio) harnstoffe, mehrere Verfahren zu ihrer Herstellung und ihre Verwendung als Schädlingsbekämpfungsmittel, insbesondere als Insektizide und Akarizide.

Es ist bereits bekannt, daß bestimmte Benzoylharnstoffe insektizide Eigenschaften besitzen (vgl. z. B. US-PS 4 139 636 oder US-PS 3 748 356). Auch die insektiziden Eigenschaften bestimmter Benzoylharnstoffe, deren Phenylteil durch eine Fluoralkylendioxy-Gruppe substituiert ist, wurden bekannt (vgl. EP-A-0 042 533, EP-A-0 097 862 und EP-A-O 093 977).

Es wurden die neuen substituierten 1-Phenyl-3-benzoyl(thio) harnstoffe der allgemeinen Formel (I)

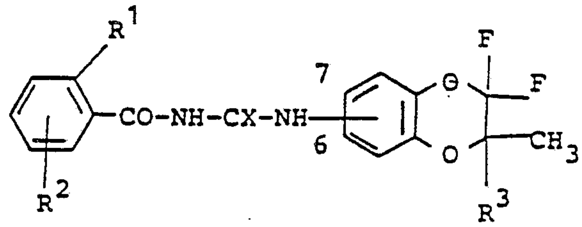

(I)

gefunden,
in welcher

X für Sauerstoff oder Schwefel steht,
$R^1$ für Halogen oder $C_1$-$C_6$-Alkyl steht,
$R^2$ für Wasserstoff, Halogen oder $C_1$-$C_6$-Alkyl steht und
$R^3$ für Wasserstoff oder Methyl steht.

Diese neuen Verbindungen weisen starke biologische, insbesondere insektizide Eigenschaften auf, die ihre Verwendung als Schädlingsbekämpfungsmittel, insbesondere als Insektizide und Akarizide ermöglichen.

Weiterhin wurde gefunden, daß die neuen substituierten 1-Phenyl-3-benzoyl-(thio) harnstoffe der allgemeinen Formel (I) erhalten werden, indem man

a) substituierte Aniline der allgemeinen Formel (II)

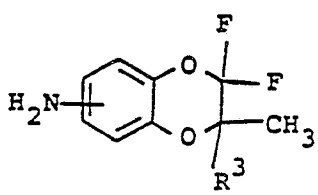

(II)

in welcher $R^3$ die oben angegebene Bedeutung hat,
mit Benzoyl-iso(thio) cyanaten der allgemeinen Formel (III)

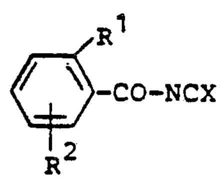

(III)

in welcher

X, $R^1$ und $R^2$ die oben angegebenen Bedeutungen haben, gegebenenfalls in Gegenwart eines Verdünnungsmittels umsetzt oder

b) substituierte Phenyl-iso(thio) cyanate der allgemeinen Formel (IV)

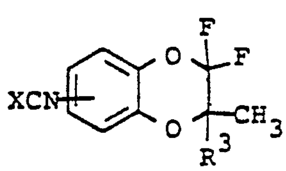

(IV)

in welcher

X und $R^3$ die oben angegebenen Bedeutungen haben, mit Benzoesäureamiden der allgemeinen Formel (V)

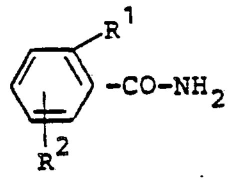

(V)

in welcher

$R^1$ und $R^2$ die oben angegebene Bedeutung haben, gegebenenfalls in Gegenwart eines Katalysators und gegebenenfalls in Gegenwart eines Verdünnungsmittels umsetzt.

Alkyl $R^1$ und $R^2$ bedeutet geradkettiges oder verzweigtes Alkyl mit 1 bis 6, vorzugsweise 1 bis 4, insbesondere 1 oder 2 Kohlenstoffatomen. Beispielhaft seien Methyl, Ethyl, n-Propyl, i-Propyl, n-Butyl, i-Butyl, sec.-Butyl und tert.-Butyl genannt.

Halogen bedeutet Fluor, Chlor, Brom und Jod, vorzugsweise Fluor, Chlor und Brom, insbesondere Fluor oder Chlor.

Der Rest $R^2$ steht vorzugsweise in der 6-Position des Phenylringes.

In den allgemeinen Formeln (I), (II) und (IV) ist der Benzodioxen-Rest vorzugsweise in 6- oder 7-Stellung an die Harnstoffgruppierung oder die $NH_2$- oder NCX-Gruppen gebunden. Die entsprechenden Verbindungen können auch als Mischungen der Verbindungen vorliegen, in denen der Benzodioxan-Rest in 6- und 7-Stellung gebunden ist.

Die neuen Verbindungen der allgemeinen Formel (I) verfügen über Eigenschaften, die ihre Verwendung als Schädlingsbekämpfungsmittel ermöglichen, insbesondere zeichnen sie sich durch eine hervorragende insektizide und akarizide Wirksamkeit aus. In überraschender Weise zeigen die neuen Verbindungen höhere insektizide Wirksamkeiten als vergleichbare Verbindungen des Standes der Technik.

Die Erfindung betrifft vorzugsweise neue Verbindungen der allgemeinen Formel (I), in welcher
X für Sauerstoff oder Schwefel steht,
$R^1$ für Fluor, Chlor, Brom oder $C_1$-$C_4$-Alkyl steht,
$R^2$ für Wasserstoff, Fluor, Chlor, Brom oder $C_1$-$C_4$-Alkyl steht und
$R^3$ für Wasserstoff oder Methyl steht.

Ganz besonders bevorzugt sind Verbindungen der allgemeinen Formel I, in welcher
X für Sauerstoff oder Schwefel steht,
$R^1$ für Fluor oder Chlor steht,
$R^2$ für Fluor oder Chlor in der 6-Stellung steht, und
$R^3$ für Methyl steht.

Verwendet man nach Verfahrensvariante (a) 2,2-Difluor-3,3-dimethyl-6-amino-1,4-benzdioxan und 2-Chlorbenzoylisocyanat als Ausgangsstoffe, so kann der Reaktionsablauf durch das folgende Formelschema wiedergegeben werden :

a)

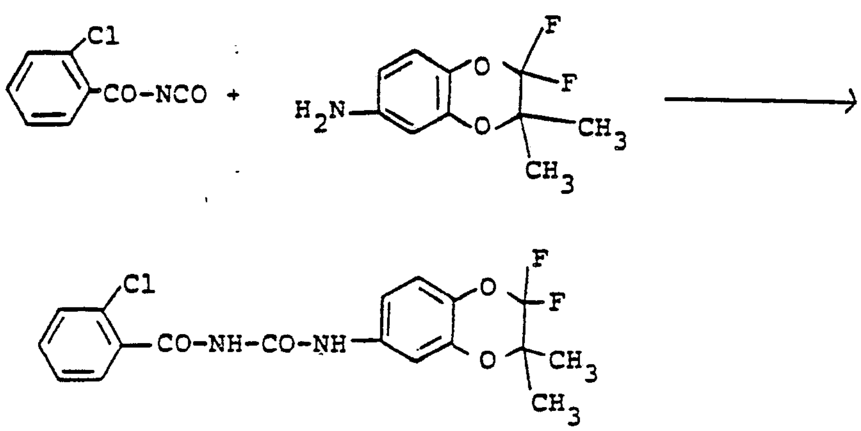

Verwendet man nach Verfahrensvariante (b) 2,2-Difluor-3,3-dimethyl-1,4-benzodioxen-6-isocyanat und 2-Chlorbenzamid als Ausgangsstoffe, so kann der Reaktionsablauf durch das folgende Formelschema wiedergegeben werden :

b)

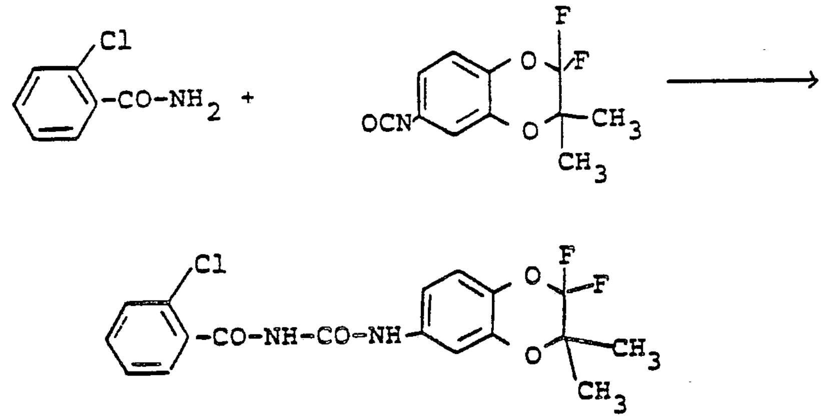

Die Ausgangsverbindungen der allgemeinen Formeln (II) und (IV) sind neu. Ihre Herstellungsverfahren werden weiter unten beschrieben.

Die Ausgangsverbindungen der Formel (III) sind bekannt oder können nach allgemein bekannten Methoden erhalten werden.

Als Beispiele für die Verbindungen der allgemeinen Formel (III) seien genannt : 2-Fluor, 2-Chlor-, 2-Brom-, 2-Methyl-, 2,6-Difluor-, 2,6-Dichlor-, 2-Chlor-6-fluor-, 2-Chlor-4-methyl, 2,6-Difluor-, 2,4-Dichlor- und 2,6-Dimethylbenzoylisocyanat bzw. -benzoylisothiocyanat.

Die Ausgangsverbindungen der allgemeinen Formel (V) sind bekannt oder können nach allgemein bekannten Methoden erhalten werden.

Als Beispiele für die Verbindungen der Formel (V) seien genannt : 2-Fluor-, 2-Chlor-, 2-Brom-, 2-Methyl-, 2,6-Difluor-, 2,6-Dichlor-, 2-Chlor-6-fluor-, 2-Chlor-4-methyl, 2,4-Difluor-, 2,5-Dichlor- und 2,6-Dimethylbenzoesäureamid.

Als Verdünnungsmittel kommen bei den Verfahrensvarianten a) und b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methyl-ethyl-, Methylisopropyl- und Methylisobutylketon, Ester, wie Essigsäuremethyl-ester und -ethylester, Nitrile, wie z. B. Acetonitril und Propionitril, Amide, wie z. B. Dimethylacetamid und N-Methyl-pyrrolidon, sowie Tetramethylensulfon.

Als Katalysatoren können für die Umsetzung gemäß Verfahrensvariante (b) vorzugsweise tertiäre Amine, wie Triethylamin und 1,4-Diazabicyclo [2,2,2]-octan, sowie organische Zinn-Verbindungen, wie z. B. Dibutylzinndilaurat verwendet werden. Das Verfahren kann jedoch auch ohne solche Katalysatoren durchgeführt werden.

Die Reaktionstemperatur kann bei beiden Verfahrensvarianten innerhalb eines größeren Bereiches variiert werden. Im allgemeinen arbeitet man bei der Verfahrensvariante a) zwischen 20 und 180 °C, vorzugsweise zwischen 40 und 120 °C und bei der Verfahrensvariante b) zwischen 20 und 200 °C, vorzugsweise zwischen 60 und 190 °C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponente bringt keine wesentlichen Vorteile.

Die Aufarbeitung der Reaktionsprodukte geschieht nach üblichen Methoden, z. B. durch Absaugen des ausgefallenen Produktes oder durch Herauslösen von unerwünschten Nebenprodukten aus dem Reaktionsgemisch. Zur Charakterisierung dient der Schmelzpunkt.

Die Verbindungen der allgemeinen Formeln (II) und (IV) sind neu. Sie können unter der allgemeinen Formel (VI) zusammengefaßt werden :

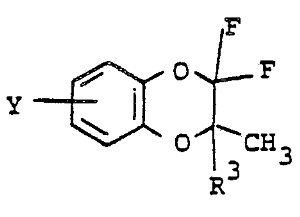

(VI)

in welcher

Y für $NH_2$ oder NCX steht, wobei
X Sauerstoff oder Schwefel bedeutet, und
$R^3$ für Wasserstoff oder Methyl steht.
Diese Verbindungen sowie Verfahren zu ihrer Herstellung sind Teil der vorliegenden Erfindung.
Die Verbindungen der allgemeinen Formel (II)

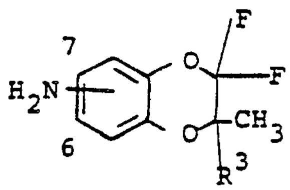

(II)

in welcher
$R^3$ für Wasserstoff oder Methyl steht,
werden dadurch erhalten, daß man die Verbindungen der allgemeinen Formel (VII) (vgl. EP-A-0 172 153)

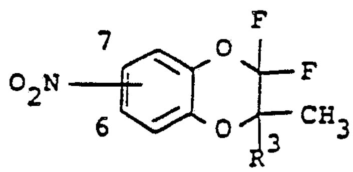

(VII)

in welcher
$R^3$ die oben angegebene Bedeutung hat,
in üblicher Weise reduziert, vorzugsweise durch katalytische Hydrierung oder mit Hilfe von Metallen oder Metallsalzen.

Die Stellungsisomeren können durch die üblichen Trennmethoden, vorzugsweise durch Destillation getrennt und isoliert werden.

Auf diese Weise können erhalten werden :

2,2-Difluor-3,3-dimethyl-6-amino-1,4-benzodioxen
" " " " -7- " " "
" " " " -5- " " "
" " " " -8- " " "
2,2-Difluor-3-methyl-6-amino-1,4-benzodioxen
" " " " -7- " " "
" " " " -5- " " "
" " " " -8- " " "

Aus den so erhaltenen Verbindungen der allgemeinen Formel (II) werden die Verbindungen der allgemeinen Formel (IV) durch die Umsetzung mit Phosgen bzw. Thiophosgen in üblicher Weise erhalten.

Für die katalytische Hydrierung der Nitroverbindungen der allgemeinen Formel (VII) kommen alle üblichen Katalysatoren in Frage, wie Raney-Nickel, Platin, Platinoxid und Palladium, wobei die Katalysatoren auch auf Trägern, z. B. Aktivkohle oder Aluminiumoxid vorliegen können.

Als Lösungsmittel kommen alle bei Hydrierungen üblicherweise eingesetzten Lösungsmittel in Frage, wie Alkohole, z. B. Methanol, Kohlenwasserstoffe, wie Toluol, oder Ether, z. B. Dioxan und Tetrahydrofuran.

Im allgemeinen setzt man erhöhte Wasserstoffdrücke, vorzugsweise zwischen 10 und 80 bar ein. Die Hydrierung wird vorzugsweise bei Temperaturen von 10 bis 100, insbesondere 20 bis 80 °C durchgeführt. Die Aufarbeitung und Isolierung der Verbindungen der allgemeinen Formel (II) erfolgt in üblicher Weise.

Die Reduktion der Nitroverbindungen der allgemeinen Formel (VII) kann in üblicher Weise auch mit Metallen wie Eisen und Zinn und deren Salzen, vorzugsweise $SnCl_2$, vorgenommen werden. Die Reduktion mit $SnCl_2$ erfolgt vorzugsweise in einem Gemisch aus wäßriger HCl und Dioxan (oder auch wasserfrei in Alkoholen, wie Ethanol) bei Temperaturen zwischen 20 und 100 °C. Analog kann auch Eisen in Form von Eisenpulver oder von Eisenspänen verwendet werden, wobei vorzugsweise Temperaturen von 80 bis 100 °C eingesetzt werden. Die Aufarbeitung und Isolierung der Verbindungen der allgemeinen Formel (II) erfolgt auch hierbei in üblicher Weise.

Die Verbindungen der allgemeinen Formel (IV) können nach den üblichen Phosgenierungsmethoden

leicht aus den Verbindungen der allgemeinen Formel (II) und Phosgen bzw. Thiophosgen erhalten werden. Als Lösungsmittel werden vorzugsweise gegebenenfalls halogenierte aromatische oder araliphatische Lösungsmittel wie Chlortoluol, Toluol oder Xylol verwendet.

Die Phosgenierung erfolgt vorzugsweise bei Temperaturen zwischen 0 und 150, insbesondere zwischen 0 und 130 °C.

Die Aufarbeitung und Isolierung der Verbindungen der allgemeinen Formel (IV) wird nach allgemein üblichen Methoden vorgenommen.

Die Herstellung der als Ausgangsmaterialien eingesetzten Verbindungen der allgemeinen Formel (VII) wird im experimentellen Teil beschrieben.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z. B. Oniscus asellus, Armadillidium vulgare, Porcellio scaber.

Aus der Ordnung der Diplopoda z. B. Blaniulus guttulatus. Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina. Aus der Ordnung der Collembola z. B. Onychiurus armatus. Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.. Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tanabus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabiltät auf gekälkten Unterlagen aus.

Die erfindungsgemäßen Wirkstoffe eignen sich auch zur Bekämpfung von unerwünschten Schädlingen auf dem Gebiet der Tierhaltung und Viehzucht, wobei durch die Bekämpfung der Schädlinge bessere Ergebnisse, z. B. höhere Milchleistungen, höheres Gewicht, schöneres Tierfell, längere Lebensdauer usw. erreicht werden können.

Die Anwendung der erfindungsgemäßen Wirkstoffe geschieht auf diesen Gebieten in bekannter Weise, wie durch äußerliche Anwendung in Form beispielsweise des Tauches (Dippen), Sprühens (Sprayen) und Aufgießens (pour-on and spot-on).

Die Herstellung der erfindungsgemäßen Verbindungen der Formel (I) soll durch die folgenden Herstellungsbeispiele erläutert werden (Verhältnisangaben bzw. Prozentangaben beziehen sich auf Gewichtsverhältnisse bzw. Gewichtsprozente) :

Beispiel 1

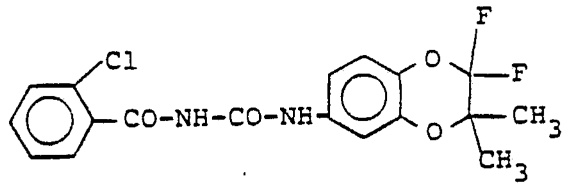

2,15 g (0,01 Mol) 2,2-Difluor-3,3-dimethyl-6-amino-1,4-benzdioxen werden in 40 ml trockenem Toluol gelöst. Bei 60 °C tropft man dazu eine Lösung von 1,82 g (0,01 Mol) 2-Chlorbenzoylisocyanat in 10 ml trockenem Toluol. Man rührt den Ansatz 0,5 Stunden bei 80 °C und kühlt ihn dann auf Raumtemperatur ab. Nach der Zugabe von etwas Petrolether wird der gebildete Niederschlag abgesaugt. Man erhält 3,2 g (Ausbeute : 80,5 % d. Th.) 1-(2,2-Difluor-3,3-dimethyl-benzo-1,4-dioxen-6-yl)-3-(2-chlorbenzoyl)-harnstoff mit einem Schmelzpunkt von 176 °C.

Analog Beispiel 1 können die Verbindungen der folgenden Beispiele hergestellt werden (je nach eingesetzten Ausgangsverbindungen stellen die Verbindungen der vorliegenden Beispiele Gemische aus den 6- und 7-Isomeren dar) :

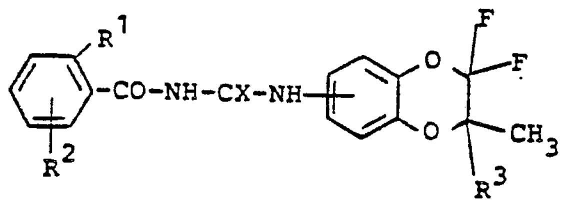

(I)

| Beispiel Nr. | $R^1$ | $R^2$ | $R^3$ | X | Fp (°C) | Ausbeute (% der Theorie) |
|---|---|---|---|---|---|---|
| 2 | Cl | 6-F | $CH_3$ | O | 185 | 67,5 |
| 3 | F | 6-F | $CH_3$ | O | 185 | 65,5 |
| 4 | Cl | 6-F | $CH_3$ | S | 211 | 79,0 |
| 5 | Br | H | $CH_3$ | S | 171 | 74,5 |
| 6 | F | 6-F | $CH_3$ | S | 211 | 91,5 |
| 7 | Cl | 6-Cl | $CH_3$ | S | 204 | 71,5 |
| 8 | Cl | 4-F | $CH_3$ | O | 210 | 91,0 |
| 9 | Cl | 4-F | $CH_3$ | S | 190 | 69,5 |
| 10 | $CH_3$ | H | $CH_3$ | S | 140 | 69,0 |

Die Herstellung der erfindungsgemäßen Verbindungen der allgemeinen Formeln (II) und (IV) soll anhand der folgenden Beispiele erläutert werden :

Beispiel 1A

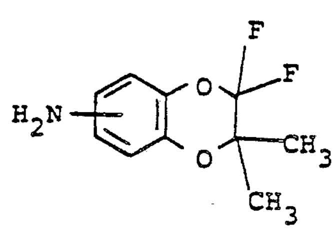

Eine Mischung von 75 g 2,2-Difluor-3,3-dimethyl-6-nitro-1,4-benzodioxen und 2,2-Difluor-3,3-dimethyl-7-nitro-1,4-benzodioxen (Verhältnis 80,5 : 19,5) wird in 400 ml Methanol in Gegenwart von 10 g Raney-Nickel bei 25-45 °C und einem Wasserstoffdruck von 10-50 bar bis zum Ende der Wasserstoff-Aufnahme hydriert. Nach Abtrennen des Katalysators durch Filtration und Abdestillieren des Methanols wird das Amin-Gemisch destilliert (Kp : 90-5 °C/0,3 mbar, $n_D^{25}$ : 1,5150), Ausbeute 55 g.

Das Gemisch läßt sich durch präparative Gaschromatographie trennen. Nach spektroskopischen Befunden handelt es sich bei der Hauptkomponente um das 6-Amino-Isomere.

7-Amino-2,2-difluor-3,3-dimethyl-1,4-benzodioxen Kp : 72-5 °C/0,05 mbar, $n_D^{20}$ : 1,5142

6-Amino-2,2-difluor-3,3-dimethyl-1,4-benzodioxen Kp : 118-20 °C/1,5 mbar, F : 54-6 °C

Beispiel 2A

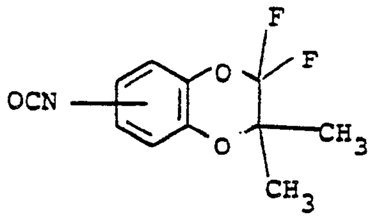

Man legt 50 g 2,2-Difluor-3,3-dimethyl-6-amino-1,4-benzodioxen (80 %ig mit 20 % 7-Amino-Isomerenanteil) in 500 ml trockenem Chlorbenzol bei ~ 0-5 °C vor und leitet ca. 40 g Phosgen ein. Dann steigert man die Temperatur kontinuierlich während der Phosgenierung bis auf zuletzt 130 °C. Dann wird mit Stickstoff ausgeblasen und anschließend fraktioniert destilliert. Man erhält 48 g 2,2-Difluor-3,3-dimethyl-6-isocyanato-1,4-benzodioxen (80 %ig mit 20 % 7-Isocyanato-Isomerenanteil) Kp : 80-5 °C/0,25 mbar.

Beispiel 3A

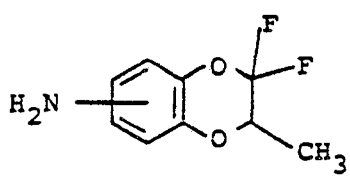

Analog Beispiel 1A erhält man aus 50 g 2,2-Difluor-3-methyl-6-nitro-1,4-benzodioxen (62 %ig + 7-Nitro-Isomerenanteil) 39 g Amino-Verbindung entsprechender Isomerenreinheit Kp : 84-88 °C/0,2 mbar.

6-Amino-2,2-difluor-3-methyl-1,4-benzodioxen Kp : 86-90 °C/0,5 mbar, F : 48 °C aus Cyclohexan 8-Amino-2,2-difluor-3-methyl-1,4-benzodioxen Kp : 80-2 °C/0,4 mbar, $n^{20}_D$ : 1,5130

Beispiel 4A

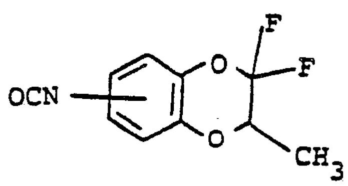

In 200 ml trockenem Chlorbenzol werden 30 g Phosgen bei 0-10 °C kondensiert und anschließend unter weiteren Einleiten von Phosgen 30 g 2,2-Difluor-3-methyl-6-amino-1,4-benzodioxen (62 %ig + 7-Amino-Isomerenanteil) in 50 ml Chlorbenzol zugetropft. Nach Ende der Zugabe wird noch bei bis 130 °C steigender Temperatur phosgeniert, anschließend mit Stickstoff ausgeblasen und destilliert. Kp : 78-81 °C/0,3 mbar.

Die Herstellung der Ausgangsverbindungen der allgemeinen Formel (VII) kann gemäß den folgenden Beispielen erfolgen :

Beispiel 1B

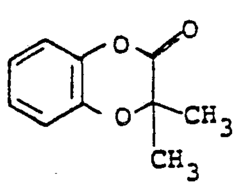

In 250 ml Acetonitril werden 70 g Kaliumcarbonat und 55 g Brenkatechin vorgelegt und auf 50 °C

erhitzt. Dann werden 100 g Bromisobuttersäuremethylester zugetropft und für 5 Stunden am Rückfluß gekocht und gerührt. Nach dem Abdestillieren des Acetonitrils werden zu dem verbliebenen Rückstand 200 ml Wasser gegeben, mit Salzsäure sauergestellt und mit Dichlormethan extrahiert. Durch Destillation werden aus dem Extrakt 74 g 2,2-Dimethyl-benzodioxen-3-on mit einem Siedepunkt von 115 bis 120 °C bei 14 mbar und einem Schmelzpunkt von 42 bis 44 °C erhalten.

Beispiel 2B

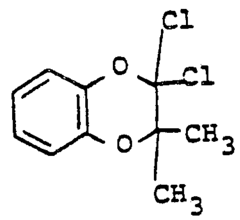

115 g 2,2-Dimethyl-benzodioxen-3-on, erhalten gemäß Beispiel 1B, werden mit 172 g Phosphorpentachlorid vermischt und unter Rühren bis zum Rückfluß erhitzt. Bei 65 °C wird die Mischung flüssig und bei 120 °C die Rückflußtemperatur erreicht. Nach 2 Stunden wird das entstandene Phosphoroxychlorid abdestilliert und das Rohprodukt einer Destillation unterzogen. Es werden 128 g 2,2-Dimethyl-3,3-dichlor-benzodioxen mit einem Siedepunkt von 132 bis 140 °C bei 20 mbar und einem Schmelzpunkt von 63 bis 64 °C (umkristallisiert aus Hexan), erhalten.

Beispiel 3B

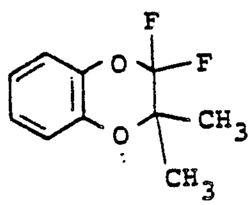

In einer Fluorierungsapparatur aus Edelstahl werden 128 g 2,2-Dimethyl-3,3-dichlor-benzodioxen, erhalten gemäß Beispiel 2B, vorgelegt und bei — 10 °C 250 ml Fluorwasserstoff zudosiert. Unter Rühren wird die Temperatur langsam auf + 10 °C gesteigert und zuletzt auf 20 °C erwärmt. Es wird gerührt bis keine Chlorwasserstoffentwicklung mehr zu beobachten ist (etwa 4 Stunden). Dann wird der überschüssige Fluorwasserstoff abdestilliert und anschließend 83 g 2,2-Dimethyl-3,3-difluor-benzo-dioxen mit einem Siedepunkt von 84 bis 86 °C bei 22 mbar und einem Brechungsindex von $n^{20}_D$ von 1,4740 erhalten.

Beispiel 4B

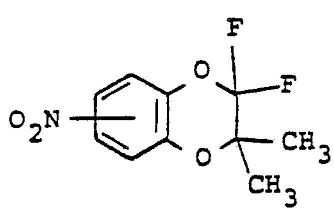

Bei 5 °C werden 70 g 2,2-Difluor-3,3-dimethyl-1,4-benzodioxen vorgelegt und eine Mischung aus 53 ml Salpetersäure (65 %ig) und 60 ml konz. Schwefelsäure zugetropft.

Danach wird 1 Stunde bei 10 °C und 1 Stunde bei 20 °C gerührt. Nachdem der Ansatz auf Eis ausgetragen wurde, werden die Phasen mit Dichlormethan getrennt, die organische Phase mit Wasser gewaschen, getrocknet und destilliert. Man erhält 76 g Nitroverbindung (Kp : 95-100 °C/0,2 mbar,$n^{20}_D$ : 1.5205), bei der Anteil des 6-Nitro-isomeren bei 80,5 % liegt.

Die Ausgangsverbindungen, in welchen $R^3$ für Wasserstoff steht, werden in analoger Weise hergestellt.

Die biologische Wirksamkeit der erfindungsgemäßen Verbindungen der allgemeinen Formel (I) soll durch das folgende biologische Beispiel erläutert werden :

Beispiel A

Plutella-Test
Lösungsmittel : 15 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden ; 0 % bedeutet, daß keine Raupen abgetötet wurden.

Bei einer Wirkstoffkonzentration von 0,1 % zeigten beispielsweise die Verbindungen der Beispiele 1 bis 6 nach 7 Tagen eine Abtötung von 100 %.

**Patentansprüche**

1. Verbindungen der allgemeinen Formel (I)

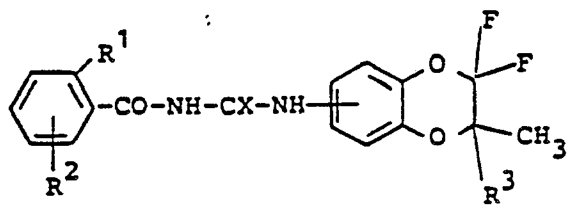

(I)

in welcher
X für Sauerstoff oder Schwefel steht,
$R^1$ für Halogen oder $C_1$-$C_6$-Alkyl steht,
$R^2$ für Wasserstoff, Halogen oder $C_1$-$C_6$-Alkyl steht und
$R^3$ für Wasserstoff oder Methyl steht.

2. Verfahren zur Herstellung der Verbindungen der allgemeinen Formel (I)

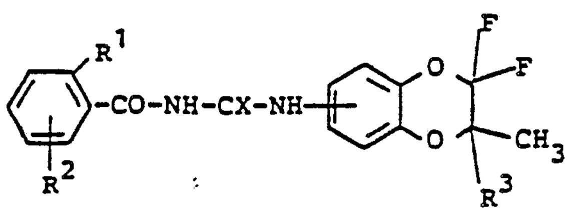

(I)

in welcher
X für Sauerstoff oder Schwefel steht,
$R^1$ für Halogen oder $C_1$-$C_6$-Alkyl steht,
$R^2$ für Wasserstoff, Halogen oder $C_1$-$C_6$-Alkyl steht und
$R^3$ für Wasserstoff oder Methyl steht, dadurch gekennzeichnet, daß man
a) substituierte Aniline der allgemeinen Formel (II)

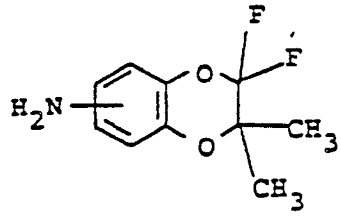

(II)

in welcher
$R^3$ die oben angegebene Bedeutung hat, mit Benzoyl-iso(thio)cyanaten der allgemeinen Formel (III)

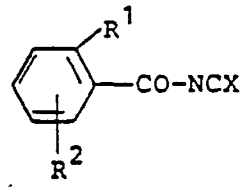

(III)

in welcher

X, $R^1$ und $R^2$ die oben angegebene Bedeutung haben, gegebenenfalls in Gegenwart eines Verdünnungsmittels umsetzt oder

b) substituierte Phenyl-iso(thio)cyanate der allgemeinen Formel (IV)

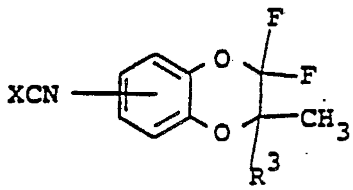

(IV)

in welcher

X und $R^3$ die oben angegebene Bedeutung haben, mit Benzoesäureamiden der allgemeinen Formel (V)

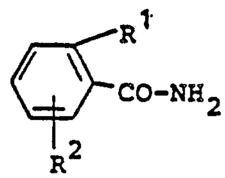

(V)

in welcher

$R^1$ und $R^2$ die oben angegebenen Bedeutungen haben, gegebenenfalls in Gegenwart eines Katalysators und gegebenenfalls in Gegenwart eines Verdünnungsmittels umsetzt.

3. Schädlingsbekämpfungsmittel, gekennzeichnet durch einen Gehalt an mindestens einer Verbindung der Formel I gemäß den Ansprüchen 1 oder 2.

4. Verwendung von Verbindungen der Formel I gemäß den Ansprüchen 1 oder 2 zur Bekämpfung von Schädlingen, insbesondere Insekten und Spinnentieren.

5. Verfahren zur Bekämpfung von Schädlingen, dadurch gekennzeichnet, daß man Verbindungen der Formel I gemäß den Ansprüchen 1 oder 2 auf die Schädlinge, vorzugsweise Insekten oder Spinnentiere oder ihren Lebensraum einwirken läßt.

6. Verfahren zur Herstellung von Schädlingsbekämpfungsmitteln, dadurch gekennzeichnet, daß man Verbindungen der Formel I gemäß den Ansprüchen 1 oder 2 mit Streckmitteln und/oder oberflächenaktiven Mitteln vermischt.

7. Verbindungen der allgemeinen Formel (VI)

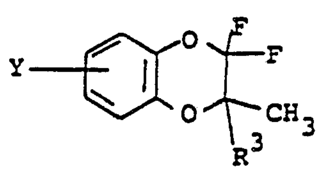

(VI)

in welcher

Y für $NH_2$ oder NCX steht, wobei X Sauerstoff oder Schwefel bedeutet, und

$R^3$ für Wasserstoff oder Methyl steht.

8. Verfahren zur Herstellung der Verbindungen der allgemeinen Formel (VI)

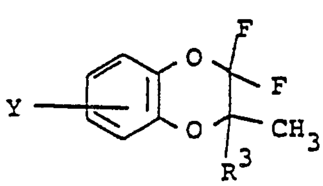

(VI)

in welcher

Y für $NH_2$ oder NCX steht, wobei X Sauerstoff oder Schwefel bedeutet, und

$R^3$ für Wasserstoff oder Methyl steht, dadurch gekennzeichnet, daß man Verbindungen der allgemeinen Formel (VII)

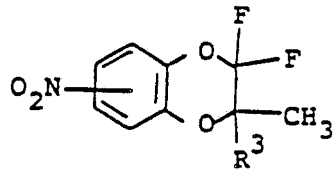

(VII)

in welcher

$R^3$ die oben angegebene Bedeutung hat, katalytisch oder mit Metallen oder Metallsalzen zu den Verbindungen der allgemeinen Formel (II)

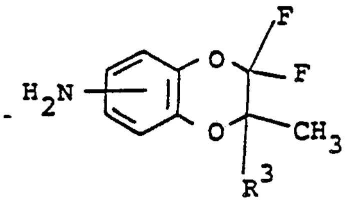

(II)

in welcher

$R^3$ die oben angegebene Bedeutung hat, reduziert und die Verbindungen der allgemeinen Formel (II) isoliert und gegebenenfalls diese Verbindungen mit Phosgen oder Thiophosgen zu den Verbindungen der allgemeinen Formel (IV)

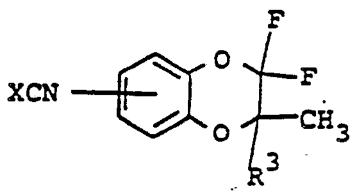

(IV)

in welcher

X und $R^3$ die oben angegebene Bedeutung haben, umsetzt.

**Claims**

1. Compounds of the general formula (I)

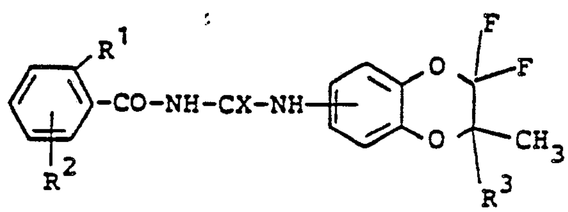

(I)

in which

X represents oxygen or sulphur,

$R^1$ represents halogen or $C_1$-$C_6$-alkyl,

$R^2$ represents hydrogen, halogen or $C_1$-$C_6$-alkyl and

$R^3$ represents hydrogen or methyl.

2. Process for the preparation of the compounds of the general formula (I)

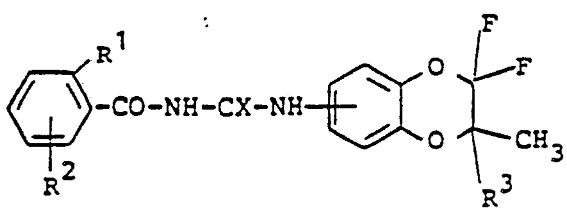

(I)

in which

X represents oxygen or sulphur,

$R^1$ represents halogen or $C_1$-$C_6$-alkyl,

$R^2$ represents hydrogen, halogen or $C_1$-$C_6$-alkyl and

$R^3$ represents hydrogen or methyl, characterised in that

a) substituted anilines of the general formula (II)

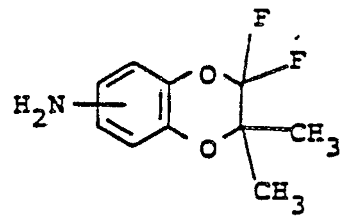

(II)

in which

$R^3$ has the abovementioned meaning, are reacted with benzoyl iso(thio)cyanates of the general formula (III)

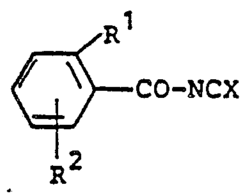

(III)

in which

X, $R^1$ and $R^2$ have the abovementioned meaning, if appropriate in the presence of a diluent, or

b) substituted phenyl iso(thio)cyanates of the general formula (IV)

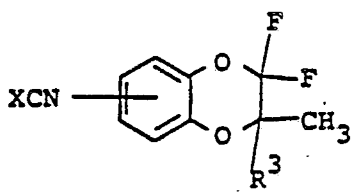

(IV)

in which

X and $R^3$ have the abovementioned meaning, are reacted with benzoic acid amides of the general formula (V)

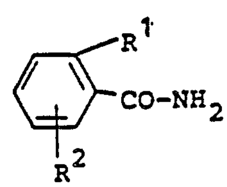

(V)

in which

$R^1$ and $R^2$ have the abovementioned meanings, if appropriate in the presence of a catalyst and if appropriate in the presence of a diluent.

3. Agents for combating pests, characterised in that they contain at least one compound of the formula I according to Claims 1 or 2.

4. Use of compounds of the formula I according to Claim 1 or 2 for combating pests, in particular insects and arachnida.

5. Method of combating pests, characterised in that compounds of the formula I according to Claim 1 or 2 are allowed to act on the pests, preferably insects or arachnida, or their environment.

6. Process for the preparation of agents for combating pests, characterised in that compounds of the formula I according to Claim 1 or 2 are mixed with extenders and/or surface-active agents.

7. Compounds of the general formula (VI)

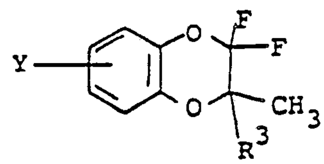

(VI)

in which

Y represents $NH_2$ or NCX,

wherein

X denotes oxygen or sulphur, and

$R^3$ represents hydrogen or methyl.

8. Process for the preparation of the compounds of the general formula (VI)

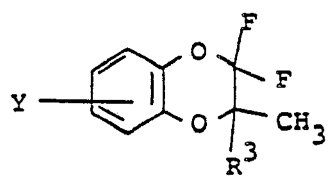

(VI)

in which

Y represents $NH_2$ or NCX,

wherein

X denotes oxygen or sulphur, and

$R^3$ represents hydrogen or methyl, characterised in that compounds of the general formula (VII)

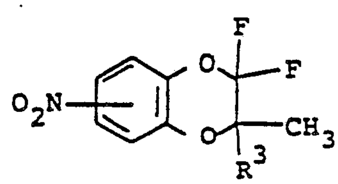

in which

$R^3$ has the abovementioned meaning, are reduced catalytically or with metals or metal salts to give the compounds of the general formula (II)

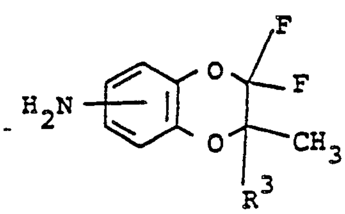

(VII)

in which

$R^3$ has the abovementioned meaning, and the compounds of the general formula (II) are isolated and, if appropriate, these compounds are reacted with phosgene or thiophosgene to give the compounds of the general formula (IV)

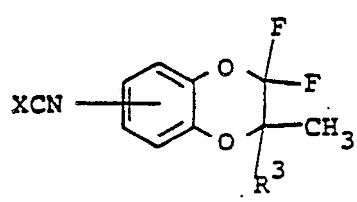

(IV)

in which

X and $R^3$ have the abovementioned meaning.

**Revendications**

1. Composés de formule générale (I)

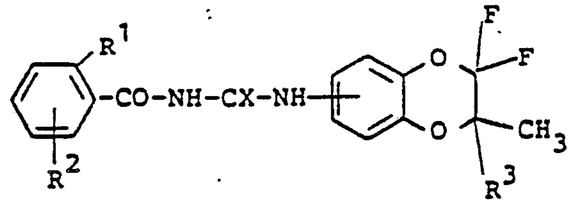

(I)

dans laquelle
X représente de l'oxygène ou du soufre,
$R^1$ représente de l'halogène ou un alcoyle en $C_1$-$C_6$,
$R^2$ représente de l'hydrogène, de l'halogène ou un alcoyle en $C_1$-$C_6$ et
$R^3$ représente de l'hydrogène ou un méthyle.

2. Procédé de fabrication des composés de formule générale (I)

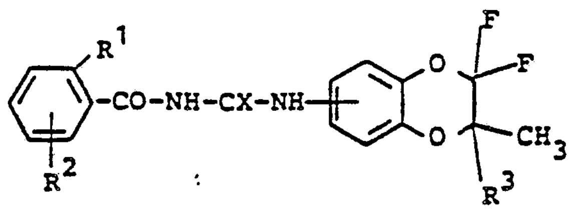

(I)

dans laquelle
X représente de l'oxygène ou du soufre,
$R^1$ représente de l'halogène ou un alcoyle en $C_1$-$C_6$,
$R^2$ représente de l'hydrogène, de l'halogène ou un alcoyle en $C_1$-$C_6$ et
$R^3$ représente de l'hydrogène ou un méthyle, caractérisé en ce que
a) on fait réagir des anilines substituées de formule générale (II)

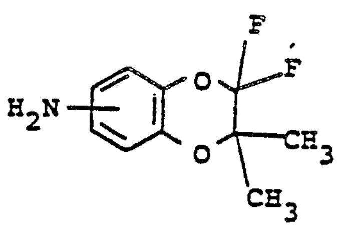

(II)

dans laquelle
$R^3$ a la signification donnée plus haut, avec des benzoyl-iso(thio)cyanates de formule générale (III) dans laquelle

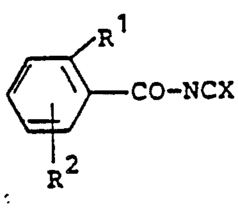

(III)

dans laquelle
X, $R^1$ et $R^2$ ont la signification indiquée plus haut, éventuellement en présence d'un diluant, ou bien
b) on fait réagir des phényl-iso(thio)cyanates substitués de formule générale (IV)

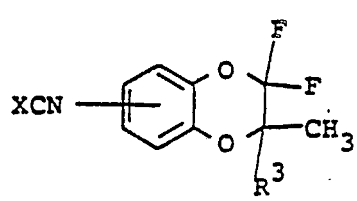

(IV)

dans laquelle
X et $R^3$ ont la signification indiquée plus haut, avec des benzamides de formule générale (V)

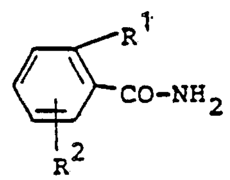

(V)

dans laquelle

$R^1$ et $R^2$ ont la signification indiquée plus haut, éventuellement en présence d'un catalyseur et éventuellement en présence d'un diluant.

3. Agents antiparasitaires, caractérisés par une teneur d'au moins un composé de formule (I) selon les revendications 1 ou 2.

4. Utilisation des composés de formule (I) selon les revendications 1 ou 2 pour combattre les parasites, en particulier les insectes et les acariens.

5. Procédé pour combattre les parasites, caractérisé en ce qu'on fait agir des composés de formule (I) selon les revendications 1 ou 2 sur les parasites, de préférence des insectes ou des acariens ou sur leur espace vital.

6. Procédé de fabrication d'agents antiparasitaires, caractérisé en ce qu'on mélange des composés de formule (I) selon les revendications 1 ou 2 avec des diluants et/ou des agents tensioactifs.

7. Composés de formule générale (VI)

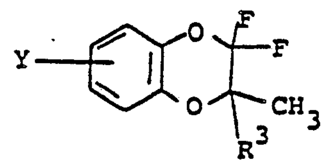

(VI)

dans laquelle

Y représente $NH_2$ ou NCX, X étant de l'oxygène ou du soufre et

$R^3$ représente de l'hydrogène ou un méthyle.

8. Procédé de fabrication des composés de formule générale (VI)

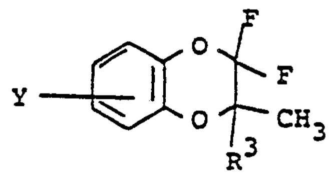

(VI)

dans laquelle

Y représente $NH_2$ ou NCX, X étant de l'oxygène ou du soufre, et

$R^3$ représente de l'hydrogène ou un méthyle, caractérisé en ce qu'on réduit des composés de formule générale (VII)

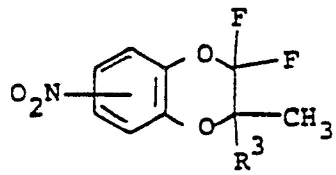

(VII)

dans laquelle

$R^3$ a la signification indiquée plus haut, catalytiquement ou avec des métaux ou sels métalliques pour donner des composés de formule générale (II)

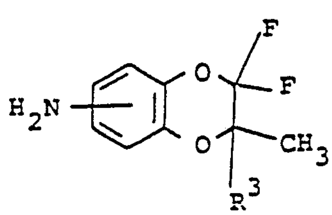

(II)

dans laquelle

$R^3$ a la signification indiquée plus haut, et en ce qu'on isole les composés de formule générale (II) et fait réagir éventuellement ces composés avec du phosgène ou du thiophosgène pour donner les composés de formule générale (IV)

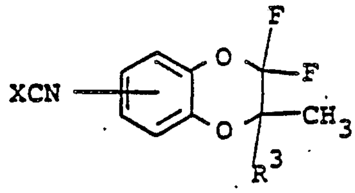

(IV)

dans laquelle

X et $R^3$ ont la signification indiquée plus haut.